# EUROPEAN PATENT APPLICATION

(11) **EP 4 659 685 A1**
(43) Date of publication of application: **10.12.2025**
(21) Application number: 24180639.7
(22) Date of filing: 07.06.2024
(51) Int. Cl.: A61B 17/064, A61B 17/12

(54) **IMPLANTABLE CLIP BETWEEN THERAPEUTIC IMPLANT AND NATIVE TISSUE FOR PREVENTING BLOOD LEAKAGE WITH HEART AND ASSOCIATED DEVICES, SYSTEMS, AND METHODS**

(71) Applicant: Koninklijke Philips N.V., 5656 AG Eindhoven (NL)
(72) Inventor: MCPEAK, Thomas John, Eindhoven (NL)
(74) Representative: Philips Intellectual Property & Standards

(57) **Abstract**

Devices, systems, and methods for preventing leakage through a therapeutic implant are provided. The apparatus includes an implantable clip having a single length of wire. The single length of wire is shaped into a first arm, a second arm, and a loop positioned laterally between the first arm and the second arm and longitudinally proximal of the first arm and the second arm. The first arm is configured to be inserted into a therapeutic implant within a heart of a patient and the second arm is configured to be inserted into a native tissue of the heart. The first arm and the second arm are configured to urge a border of the therapeutic implant and a border of the native tissue toward one another and close a gap between the therapeutic implant and the native tissue.

## Description

### TECHNICAL FIELD

The subject matter described herein relates to stopping leakage around therapeutic implants and, in particular, leakage around therapeutic cardiac implants such as replacement valves and occlusion devices. For example, clips can be catheter-deployed to stop such leaks by closing gaps between the therapeutic implant and the anatomy.

### BACKGROUND

Various types of therapeutic implants may be implanted into the heart to treat a variety of heart conditions. For example, a heart valve may be damaged or diseased such that blood flow does not flow properly therethrough. In some cases, the valve may allow blood to flow in the opposite direction from normal blood flow (i.e. regurgitation) or significantly alter the blood flow in the direction of normal blood flow. In other cases, there may be stenosis of the valve that prevents sufficient blood flow therethrough. Thus, the existing valve may be removed and/or trimmed and a mechanical, bioprosthetic, and tissue-engineered replacement valve may be implanted in place of the existing valve. The replacement valve may be designed to replace the existing, natural valve to provide normal blood flow through the heart.

In another example, for patients with diseases like atrial fibrillation, there may be a high risk of developing blood clots in the left atrial appendage (LAA). Thus, an occlusion device may be implanted at the opening of the LAA to prevent blood flow therein, thereby minimize the risk of clots developing in the LAA.

However, when therapeutic implants such as these are implanted into a patient, there may be leakage between the exterior of the therapeutic implant and the wall of the anatomy of the heart. When a therapeutic implant is deployed in the heart, the circumference of the implant may not fully oppose the wall of the anatomy, resulting in one or more gaps between the implant and the wall. Undesirable blood flow may move through these gaps (i.e. leakage), resulting in lower performance of the implant. For example, in some cases, the therapeutic implant may not expand to the full size of the opening. This may occur because therapeutic implants are generally packaged and sold in discrete sizes, which are not tailored to the patient's specific anatomy. Thus, these gaps may form when an implant smaller than the size of the anatomy is deployed at the treatment site. In other cases, the therapeutic implant may have a circular shape and the anatomy has an ovular or non-circular shape. Thus, gaps may be created where the circumference of the circular-shaped plug and the non-circular shaped anatomy are not aligned. In either of these cases, the gaps between the therapeutic implant and the anatomy are generally non-circular and may be a variety of shapes, including crescent-shaped.

Thus, after deploying the therapeutic implant, it may be desirable (or necessary) to plug or fill these gaps to prevent leakage. Current methods of treating these gaps use circular devices or plugs. However, because the gaps are often non-circular, the circular plugs may not sufficiently plug the gap to prevent blood flow therethrough.

The information included in this Introduction section of the specification, including any references cited herein and any description or discussion thereof, is included for context and/or technical reference purposes only and is not to be regarded as subject matter by which the scope of the disclosure is to be bound or otherwise limited in any manner.

### SUMMARY

Aspects of the present disclosure are directed to a leak plugging device, with associated systems, and a method to treat leakage between a therapeutic device and the anatomy using one or more clips that have been pre-loaded into a single delivery system. The device includes a catheter-based deployment device with a clip disposed therein. An actuator may be disposed within the catheter and may engage and/or hold the clip. The catheter can be placed at a gap between a therapeutic device and a wall of the heart (e.g. location of a heart valve, LAA). The actuator and/or catheter can then be moved to push the clip out of the distal opening of the catheter and implant the a first arm of the clip into the therapeutic device and a second arm of the clip into the anatomy. The actuator and/or catheter can then be moved so that the walls of the catheter apply a force to the arms of the clip to move the arms inward towards each other, thereby crimping the clip. As the arms move inward, they pull the therapeutic device and/or anatomy closer towards each other until they are in contact or nearly in contact. In this way, the clip is configured to close the gap. In some cases, multiple clips may be required to close the gap or gaps. Because the clip pulls the therapeutic device and anatomy together, the clips can be deployed to close gaps of any shape. Thus, the present disclosure advantageously provides an implantable clip that can sufficiently close gaps of all shapes and sizes, including non-circular gaps.

In an exemplary aspect, an apparatus is provided. The apparatus includes an implantable clip having a single length of wire. The single length of wire is shaped into a first arm, a second arm, and a loop positioned laterally between the first arm and the second arm and longitudinally proximal of the first arm and the second arm. The first arm is configured to be inserted into a therapeutic implant within a heart of a patient and the second arm is configured to be inserted into a native tissue of the heart. The first arm and the second arm are configured to urge a border of the therapeutic implant and a border of the native tissue toward one another and close a gap between the therapeutic implant and the native tissue.

In one aspect, the first arm includes a barbed first end that is inserted into the therapeutic implant and the second arm comprises a barbed second end that is inserted into the native tissue. In one aspect, the apparatus also includes a delivery catheter having a lumen configured to receive the implantable clip before the first arm is inserted into the therapeutic implant and the second arm is inserted into the native tissue of the heart. In one aspect, when the implantable clip is positioned within the lumen, the first arm includes a first bend on a first side of the loop and the second arm includes a second bend on a second side of the loop. The first bend and the second bend are proximate to a first side of the delivery catheter and the loop is proximate to an opposite second side of the delivery catheter. In one aspect, the first arm includes a barbed first end proximal of the first bend and the second arm includes a barbed second end distal of the second bend. When the implantable clip is positioned within the lumen, the barbed first end is proximal-facing and the barbed second end is distal-facing. In one aspect, the apparatus further includes an actuator positioned within the lumen and configured to be removably coupled to the implantable clip. In one aspect, the actuator is shaped to be received through the loop to removably couple the actuator and the implantable clip. In one aspect, the delivery catheter includes a catheter wall defining an opening at a distal end of the lumen and the actuator is configured to provide distal motion to the implantable clip to move the implantable clip outside of the lumen.

In one aspect, the actuator is configured to provide proximal motion to the implantable clip, while the implantable clip is positioned outside of the lumen, such that the first and second arm contact the catheter wall. In response to the proximal motion, the catheter wall is configured to urge the first arm and the second arm towards one another while the first arm is inserted into the therapeutic implant and the second arm is inserted into the native tissue such that the border of the therapeutic implant and the border of the native tissue are urged toward one another. In one aspect, the therapeutic implant includes a prosthetic valve such that the gap comprises a paravalvular leakage or an occlusion device such that the gap comprises an occlusion leakage.

In an exemplary aspect, a system for treating leakage between a therapeutic device and tissue of a patient is provided. The system includes a catheter and an implant. The catheter includes a lumen, a distal portion, and a proximal portion. The implant is disposed in the lumen at the distal portion of the catheter before the implant is implanted in the patient. The implant includes a first wire including a first end having a first attachment member configured to be inserted into the therapeutic device and a second end having a second attachment member configured to be inserted into the tissue.

In one aspect, at least one of the first attachment member or the second attachment member includes a hook. In one aspect, at least one of the first attachment member or the second attachment member includes a plurality of barbs. In one aspect, the system further includes an actuator disposed within the lumen of the catheter and configured to move the implant distally within the lumen such that the implant exits the distal portion of the catheter. In one aspect, the actuator includes a second wire comprising a distal end and a bend proximate the distal end, where the bend contacts the implant. In one aspect, the bend in the second wire is approximately 90 degrees such that the second wire is L-shaped at the distal end. In one aspect, the second wire includes a first length and a second length that are equal to one another. The first length is located on a first side of the bend and the second length is located on a second side of the bend. In one aspect, the actuator is further configured to move a portion of the implant proximally within the lumen of the catheter after the first end of the first wire has been implanted in the implanted device and the second end of the first wire has been implanted in the tissue such that contact between the implant and the catheter moves the first end closer to the second end. In one aspect, the first wire is flexible such that the first wire has a first shape outside of the lumen of the catheter and a different, second shape inside of the lumen of the catheter. In one aspect, the first wire includes a loop between the first end and the second end.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of aspects of the present disclosure, e.g., as defined in the claims, is provided in the following written description of various examples and/or aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1A is a side view of a human heart according to aspects of the present disclosure.
Fig. 1B is a cross-sectional side view of a human heart according to aspects of the present disclosure.
Fig. 2 is a cross-sectional side view of an aortic valve replacement in a human heart according to aspects of the present disclosure.
Fig. 3 is a diagrammatic, cross-sectional top view of an aortic valve replacement of a degenerated natural aortic valve by a transvenous/transcatheter aortic valve repair (TAVR), according to aspects of the present disclosure.
Fig. 4A is a diagrammatic top view of a replacement valve positioned within the heart, in the open position, according to aspects of the present disclosure.
Fig. 4B is a diagrammatic top view of the replacement valve of Fig. 4A in the closed position, according to aspects of the present disclosure.
Fig. 5A is a diagrammatic cross-sectional side view of a replacement valve in an open position, according to aspects of the present disclosure.
Fig. 5B is a diagrammatic cross-sectional side views of a replacement valve in a closed position, according to aspects of the present disclosure.
Fig. 6 is a cross-sectional view of an occlusion device implanted in the left atrial appendage (LAA), according to aspects of the present disclosure.
Fig. 7A is a diagrammatic cross-sectional view of the occlusion device along section line 7-7 in Fig. 6, according to aspects of the present disclosure.
Fig. 7B is a diagrammatic cross-sectional view of the LAA along section line 7-7 in Fig. 6, according to aspects of the present disclosure.
Fig. 7C is a diagrammatic cross-sectional view of the occlusion device deployed within the LAA, according to aspects of the present disclosure.
Fig. 8 is schematic, diagrammatic view of a system according to aspects of the present disclosure.
Fig. 9A is a front diagrammatic view of the implantable clip, according to aspects of the present disclosure.
Fig. 9B is a side diagrammatic view of the implantable clip, according to aspects of the present disclosure.
Fig. 10A is a cross-sectional diagrammatic view of the clip delivery catheter and actuator without the clip, according to aspects of the present disclosure.
Fig. 10B is a cross-sectional diagrammatic view of the clip delivery catheter and actuator with the clip, according to aspects of the present disclosure.
Fig. 11A is a diagrammatic view of an aspect of an actuator, according to aspects of the present disclosure.
Fig. 11B is a diagrammatic view of another aspect of an actuator, according to aspects of the present disclosure.
Figs 12A-12G are diagrammatic views of a series of steps in a method for delivering and deploying a clip using a clip delivery catheter and actuator, according to aspects of the present disclosure.
Fig. 13A-13E are diagrammatic views of a series of steps in another method for delivering and deploying a clip using a clip delivery catheter and actuator, according to aspects of the present disclosure.
Fig. 14A is a diagrammatic cross-sectional side view of the replacement valve with a clip implanted, according to aspects of the present disclosure.
Fig. 14B is a diagrammatic cross-sectional side view of the replacement valve with a clip implanted, according to aspects of the present disclosure.
Fig. 15 is a diagrammatic cross-sectional view of an occlusion device with multiple clips implanted, according to aspects of the present disclosure.
Fig. 16 is a schematic diagram of a processor circuit, according to aspects of the present disclosure.

### DETAILED DESCRIPTION

For the purposes of promoting an understanding of the principles of the present disclosure, reference will now be made to the examples illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one example and/or aspect may be combined with the features, components, and/or steps described with respect to other examples and/or aspects of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

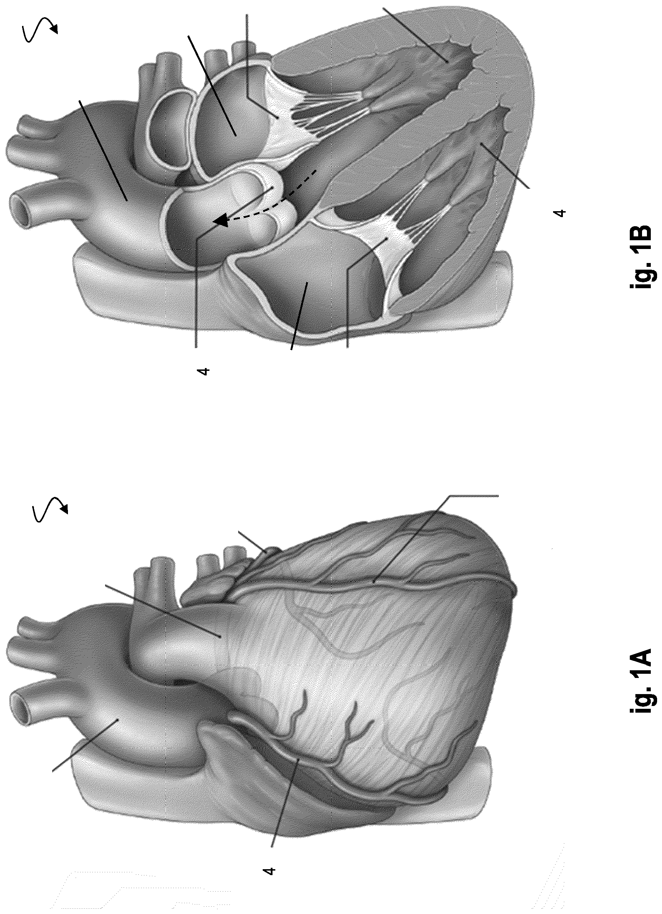

Fig. 1A is a side view of a human heart 100 according to aspects of the present disclosure. Visible are an aorta 102 from which stems a right coronary artery 104 and a left main coronary artery 106. The left main coronary artery 106 branches into a left circumflex coronary artery 108 and a left anterior descending coronary artery 110. The right coronary artery 104, the left main coronary artery 106, the left circumflex coronary artery 108, and a left anterior descending coronary artery 110 are the arteries that provide oxygen-rich blood to muscles of the human heart 100.

Fig. 1B is a cross-sectional side view of a human heart 100 according to aspects of the present disclosure. Visible are a right atrium 112 and a right ventricle 114. In that regard, oxygen-poor blood enters the human heart 100 in the right atrium 112 and travels to the right ventricle 114 through the tricuspid valve 116. The oxygen-poor blood leaves the right ventricle 114 and travels to the lungs. Also visible are a left atrium 118 and a left ventricle 120. In that regard, oxygen-rich blood is received from the lungs in the left atrium 118 and travels to the left ventricle 120 through the mitral valve 122. The oxygen-rich blood leaves the left ventricle 120 and goes out to the body through the aorta 102 via an aortic valve 124.

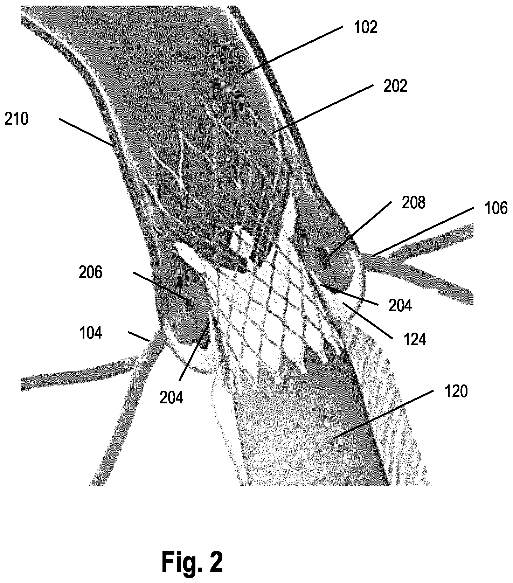

Fig. 2 is a cross-sectional side view of an aortic valve 124 replacement in a human heart 100 according to aspects of the present disclosure. In some aspects, e.g., when aortic valve stenosis has occurred to the aortic valve 124 that keeps blood flowing in the correct direction from the left ventricle 120 to the aorta 102, a transvenous/transcatheter aortic valve repair (TAVR) procedure may be performed to replace the natural aortic valve 124 with a replacement aortic valve 202. In some instances, portions of one or more leaflets 204 of the natural aortic valve 124 may be resected so that openings 206 and 208 to the right coronary artery 104 and the left main coronary artery 106, respectively, so that, when the one or more leaflets 204 are pressed against the natural heart wall 210, the openings 206 and 208 remain unobstructed so that oxygen-rich blood may flow to the muscles of the human heart. The natural heart wall 210 may be a natural aorta wall, a natural heart chamber wall, or a natural aortic valve wall.

The TAVR procedure is shown here for exemplary purposes only; it is understood that other heart valves and heart valve replacement procedure types may generate paravalvular leaks that require plugging, and thus fall within the scope of the present disclosure.

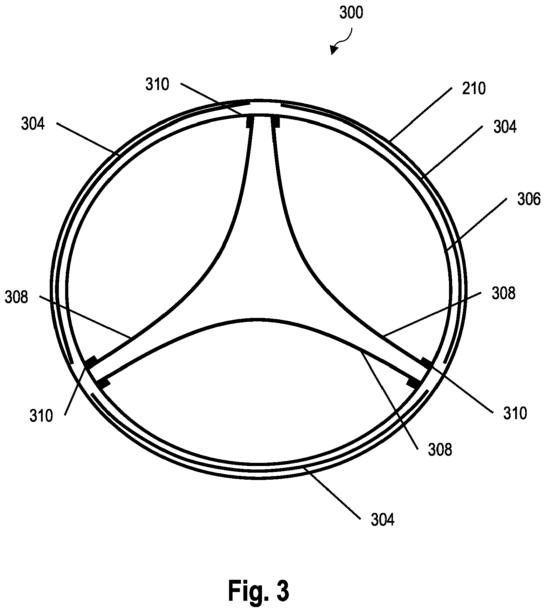

Fig. 3 is a diagrammatic, cross-sectional top view of an aortic valve replacement 300 of a degenerated natural aortic valve utilizing a TAVR valve, according to aspects of the present disclosure. Visible are the natural heart wall 210, the natural aortic valve leaflets 304, the TAVR valve wall 306, the TAVR valve leaflets 308, and the TAVR commissural tabs 310. The TAVR valve leaflets 308 may for example be constructed from bovine animal tissue coupled to the wire frame of the TAVR valve wall 306 via the commissural tabs 310. When inserted, the TAVR valve pushes the remaining unresected portions of the natural aortic valve leaflets 304 against the natural heart wall 210 such that the natural aortic valve leaflets 304 are pinned and/or secured between an outside of the TAVR valve wall 306 and the natural heart wall 210.

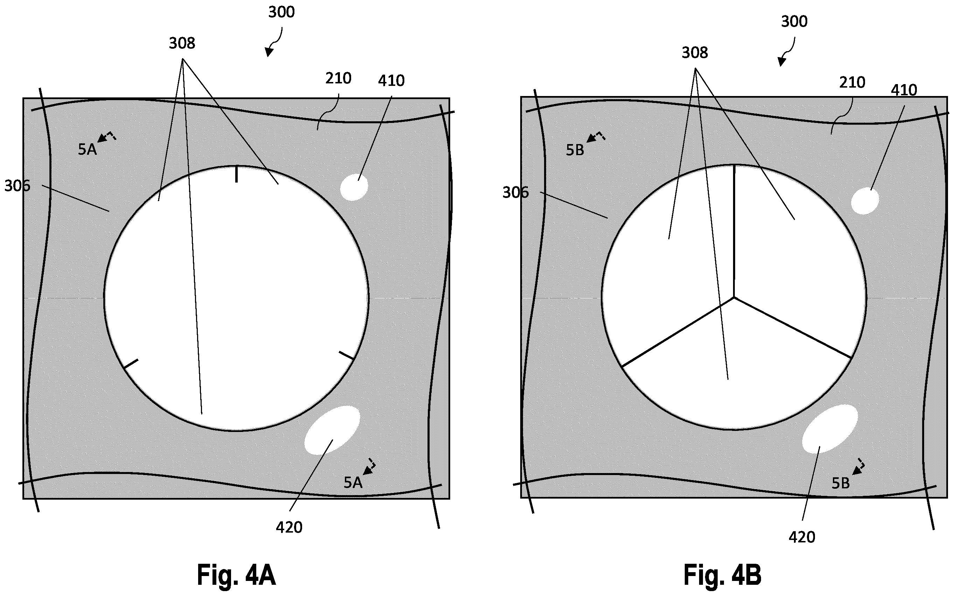

Fig. 4A is a diagrammatic top view of a replacement valve 300 (e.g., a prosthetic, mechanical, or donor valve) positioned within the heart, in the open position, according to aspects of the present disclosure. The leaflets 308 of the replacement valve periodically open (to allow blood flow in desired direction) and closed (to prevent blood flow in opposite undesired direction) during heart cycle. Artificial leaflets are one example of a mechanism that open/closes to periodically allow/stop blood flow, but a mechanical/prosthetic valve or donor valve can have any suitable mechanism that open/closes to periodically allow/stop blood flow.

In the example shown in Fig. 4A, the replacement valve wall 306 is joined to the natural heart wall 210, but it is imperfect because of a relatively smaller paravalvular leak 410 and a relatively larger paravalvular leak 420. A paravalvular leak is gap between the outer/outermost perimeter or surface of the replacement valve (e.g., a prosthetic valve cuff) and the natural heart tissue (e.g., native annulus).

Fig. 4B is a diagrammatic top view of the replacement valve 300 of Fig. 4A in the closed position, according to aspects of the present disclosure. Visible are the heart wall 210, replacement valve wall 306, leaflets 308, small paravalvular leak 410, and larger paravalvular leak 420. In the closed position, the leaflets 308 should cause blood to stop flowing through the valve. However, because blood can flow through the paravalvular leaks 410 and 420, the functioning of the replacement valve 300 may be significantly compromised.

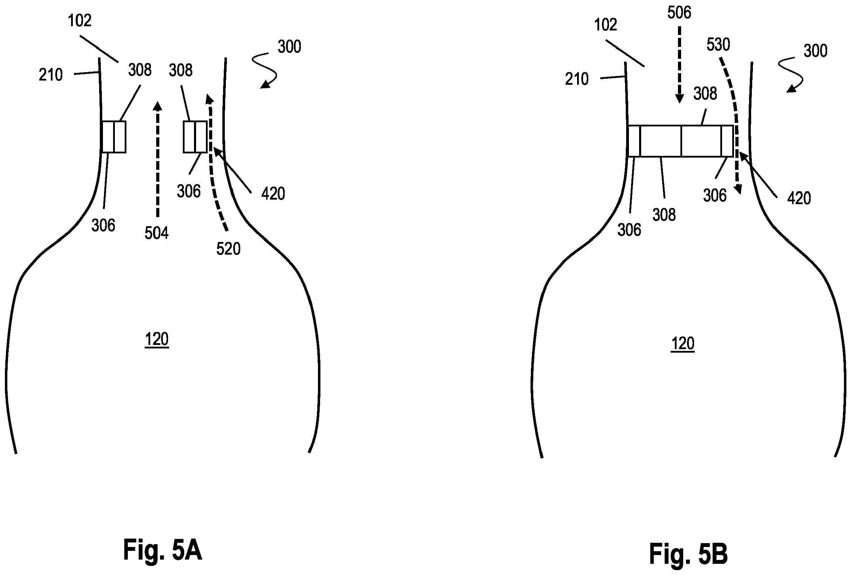

Fig. 5A is a diagrammatic cross-sectional side view of a replacement valve 300 according to aspects of the present disclosure. Visible are the natural heart wall 210 and the replacement valve leaflets 308. In the example shown in Fig. 5A, during systole (indicated by directional arrows 504), the valve leaflets 308 open and allow blood to flow from the left ventricle 120 to the aorta 102 indicated by directional arrows 504. However, paravalvular leak 420 allows leakage flow 520 to bypass the valve. Such leakage flow 520 is in the same direction as the normal blood flow 504, but may adversely impact the flow velocity, flow volume that should be entering the aorta 102.

Fig. 5B is a diagrammatic cross-sectional side views of a replacement valve 300 according to aspects of the present disclosure. Visible are the natural heart wall 210 and the replacement valve leaflets 308. In the example shown in Fig. 5B, during diastole (indicated by directional arrows 506), the valve leaflets 308 may be pushed by the flow of blood from the aorta 102 toward the left ventricle 120 such that the valve leaflets 308 close to prevent mitral regurgitation. However, the paravalvular leak 420 allows leakage flow 530 in direction 506, where blood should not be flowing at all, thus significantly compromising the function of the replacement valve 300, by effectively causing or emulating regurgitation (which may, in some cases, be a symptom for which the natural valve was replaced). Thus, to prevent such leakage flow 530, it may be critically important for the health of the patient to block or plug the paravalvular leak 420.

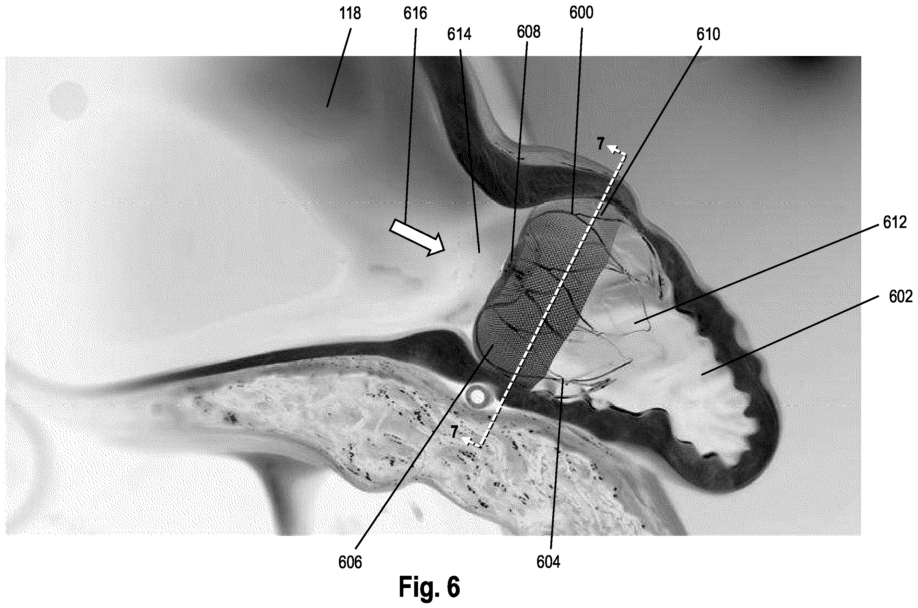

Fig. 6 is a cross-sectional view of an occlusion device 600 disposed in the left atrial appendage (LAA) of the left atrium 118 of the heart 100, according to aspects of the present disclosure. The occlusion device 600 may include a structure or cage 604 that forms an umbrella-like shape when expanded. The shape of the structure 604 may have a flat top 608 with rounded or curved sides 610 that curve around the bottom 612. The bottom 612 may be open. A mesh 606 may be disposed over and coupled to the top 608 and/or sides 610 of the structure 604. Thus, the mesh 606 may move with the structure 604 when it is expanded and/or contracted.

The occlusion device 600 may be delivered to the LAA 602 via a catheter such that the occlusion device 600 is folded within the catheter before deployment. When the distal end of the catheter is disposed proximate the opening 614 of the LAA 602, the occlusion device 600 may be deployed such that the top 608 is disposed proximate to or at the opening 614 and the bottom 612 and/or sides 610 are disposed within the LAA 602. When the occlusion device 600 exits the catheter, it may expand into the umbrella-like shape described above until the sides 610 contact the walls of the LAA 602. In some aspects, the top 608 and/or bottom 612 also contact the walls of the LAA 602. When the occlusion device 600 contacts the walls of the LAA 602, it may prevent blood flow into the LAA 602 (as shown by arrow 616), thereby minimizing the chance of blood clots developing within the LAA 602.

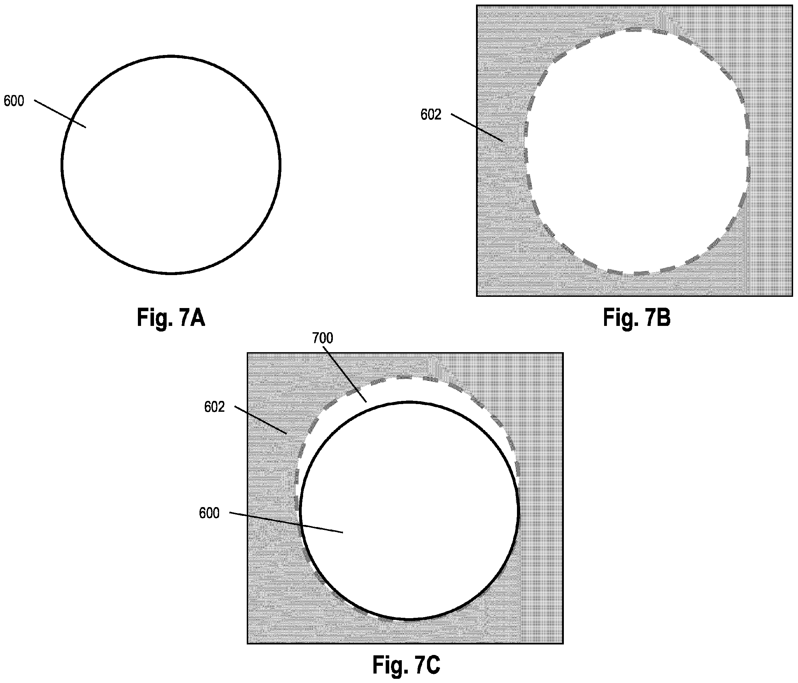

However, in some aspects, the occlusion device 600 may not completely close off the LAA 602. Figs 7A-7C illustrate an exemplary aspect in which the occlusion device 600 does not fully close off the LAA 602. Fig. 7A illustrates a diagrammatic cross-sectional view of the occlusion device 600 along the section line 7-7 in Fig. 6 from the direction indicated by the blood flow arrow 616, according to aspects of the present disclosure. This cross-section may be taken along the part of the occlusion device 600 with the largest diameter, which contacts the walls of the LAA. In the illustrated aspect, the cross-section of the occlusion device 600 is generally circular. In other aspects, the cross-section may be ovular, oblong, or any other suitable shape.

Fig. 7B illustrates a diagrammatic cross-sectional view of the walls of the LAA 602 along section line 7-7 in Fig. 6 from the direction indicated by the blood flow arrow 616, according to aspects of the present disclosure. In the illustrated aspect, the cross-section of the LAA 602 is oblong such that it is not perfectly circular. In other aspects, the cross-section may be circular, ovular, or any other suitable shape.

Fig. 7C illustrates a diagrammatic cross-sectional view of the occlusion device 600 deployed within the LAA 602 along section line 7-7 in Fig. 6 from the direction indicated by the blood flow arrow 616, according to aspects of the present disclosure. Because the cross-section of the occlusion device 600 is circular and the cross-section of the LAA 602 is non-circular, the occlusion device 600 does not continuously oppose the walls of the LAA 602. Thus, a gap 700 exists between part of the occlusion device 600 and the wall of the LAA 602. As shown in Fig. 7C, this gap 700 may be generally crescent-shaped or another non-circular shape. As described above in reference to deployment of a replacement valve 300 in Figs 5A-5B, blood flow may move through the gap between the device and the native tissue. Thus, when a gap 700 exists between the wall of the LAA 602 and the occlusion device 600, blood flow may enter and exit the LAA 602 via the gap 700. This may prevent the occlusion device 600 from fully sealing the LAA 602 as desired and may allow blood clots to form within the LAA 602.

Thus, when there are gaps (e.g. gaps 410, 420, 700) formed between the anatomy (e.g. heart wall 210 or LAA wall 602) and a therapeutic device (e.g. a replacement valve 300 or an occlusion device 600), this may prevent the therapeutic device from functioning as desired or needed. Thus, it may be advantageous to plug or close these gaps to prevent unwanted blood flow around the therapeutic device.

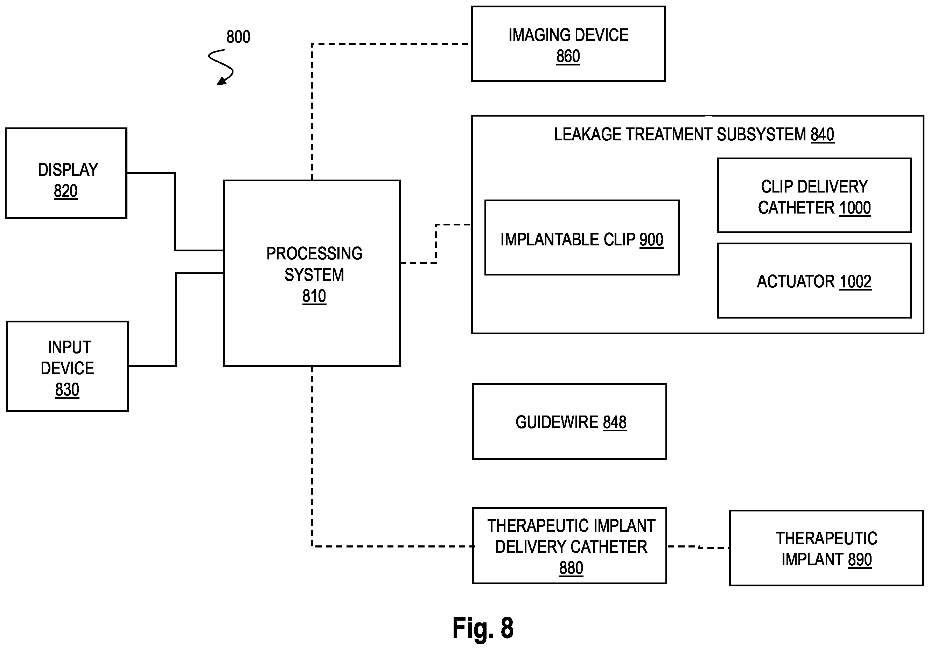

Fig. 8 is schematic, diagrammatic view of a system 800 that may prevent leakage between a therapeutic device or implant 890 and the anatomy of the patient, according to aspects of the present disclosure. The system 800 may be configured to evaluate (e.g., assess), display, and/or control (e.g., modify) one or more aspects of the delivery and/or deployment of a therapeutic implant 890 or the delivery and/or deployment of an implantable clip 900. For instance, the system 800 may be utilized to monitor and/or control one or more portions of the delivery and/or deployment of a therapeutic implant 890 or the delivery and/or deployment of an implantable clip 900. In this regard, the system 800 may be used to assess coronary vessels and/or heart tissue (e.g., the myocardium). As illustrated, the system 800 may include a processing system 810 in communication with a display device 820 (e.g., an electronic display or monitor), an input device 830 (e.g., a user input device, such as a keyboard, mouse, joystick, microphone, and/or other controller or input device), a leakage treatment subsystem 840 and/or an imaging device 860 (e.g., x-ray, computed tomography or CT, magnetic resonance imaging or MRI, etc.). As illustrated, the system 800 may further include a therapeutic implant delivery catheter 880 and a therapeutic implant 890. As explained above, the therapeutic implant 890 may be a replacement valve 300 (e.g. a prosthetic, mechanical, or donor valve) or an occlusion device 600 (e.g. for closing off the LAA 602). In some aspects, delivery and/or deployment of the therapeutic implant 890 via the therapeutic implant delivery catheter 880 may be completely mechanical (no connection to the processing system 810) or may be connected to processing system 810 (e.g., for control of movement and/or deployment of therapeutic implant). It is understood that other types of therapeutic implants and therapeutic implant delivery systems may be used instead of or in addition to those described herein.

The leakage treatment subsystem 840 includes a clip delivery catheter 1000, an actuator 1002, and an implantable clip 900 that can be deployed from the clip delivery catheter 1000 by being pushed out by the actuator 1002, as described in more detail below. In some aspects, delivery and/or deployment of the implantable clip 900 via the clip delivery catheter 1000 may be completely mechanical (no connection to the processing system 810) or may be connected to processing system 810 (e.g., for control of movement and/or deployment of clip).

Either or both of the clip delivery catheter 1000 and the therapeutic implant delivery catheter 880 may be guided over a guidewire 848.

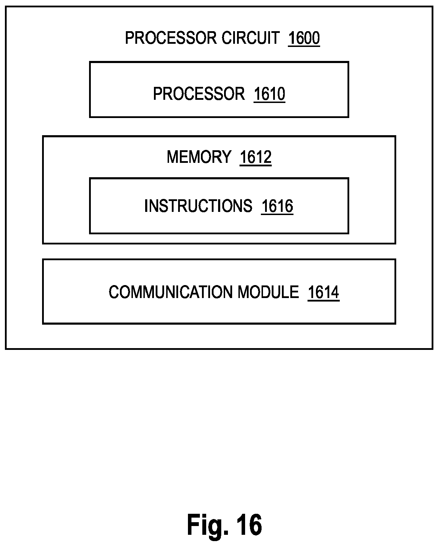

The processing system 810 is generally representative of any device suitable for performing the processing and analysis techniques disclosed herein. In some aspects, the processing system 810 includes a processor circuit, such as the processor circuit 1600 of Fig. 16, which is described in more detail below. In some aspects, the processing system 810 is programmed to execute steps associated with the data acquisition, analysis, and/or instrument (e.g., device) control described herein. Accordingly, it is understood that any steps related to data acquisition, data processing, instrument control, and/or other processing or control aspects of the present disclosure may be implemented by the processing system 810 (e.g., computing device) using corresponding instructions stored on or in a non-transitory computer readable medium accessible by the computing device. In some instances, the processing system 810 is a console device. Further, it is understood that in some instances the processing system 810 includes one or a plurality of computing devices, such as computers, with one or a plurality of processor circuits. In this regard, it is particularly understood that the different processing and/or control aspects of the present disclosure may be implemented separately or within predefined groupings using a plurality of computing devices. Any divisions and/or combinations of the processing and/or control aspects described below across multiple computing devices are within the scope of the present disclosure.

The system 800 is configured such that when the clip delivery catheter 1000 is positioned within the heart, images captured by the imaging system 860 can show the location and orientation of the clip delivery catheter 1000, and also potentially anatomical features such as the therapeutic implant 890, heart wall, and heart valve leaflets.

It is noted that block diagrams are provided herein for exemplary purposes; a person of ordinary skill in the art will recognize myriad variations that nonetheless fall within the scope of the present disclosure. For example, block diagrams may show a particular arrangement of components, subcomponents, modules, units, etc. It is understood that some aspects of the systems disclosed herein may include additional components, that some components shown may be absent from some aspects, and that the arrangement of components may be different than shown, while still performing the methods described herein.

It is understood that, in some instances, one or more components of the system 800 can operate without one or more other components of the system 800. For example, the leakage treatment subsystem 840, guidewire 848, therapeutic implant delivery catheter 880, and/or therapeutic implant 890 can be implemented without the processing system 810. For example, the leakage treatment subsystem 840, guidewire 848, therapeutic implant delivery catheter 880, and/or therapeutic implant 890 can be mechanical components that do not have signal communication with the processing system 810.

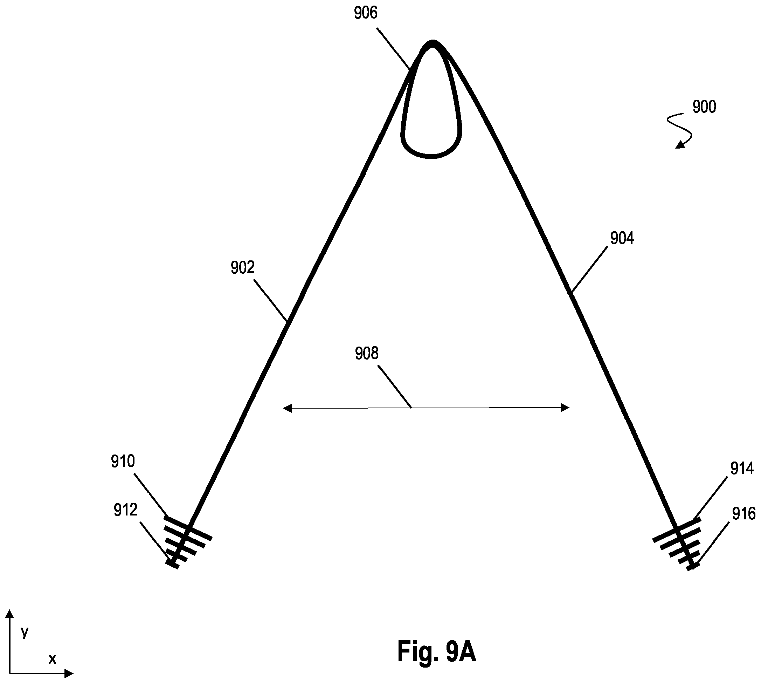

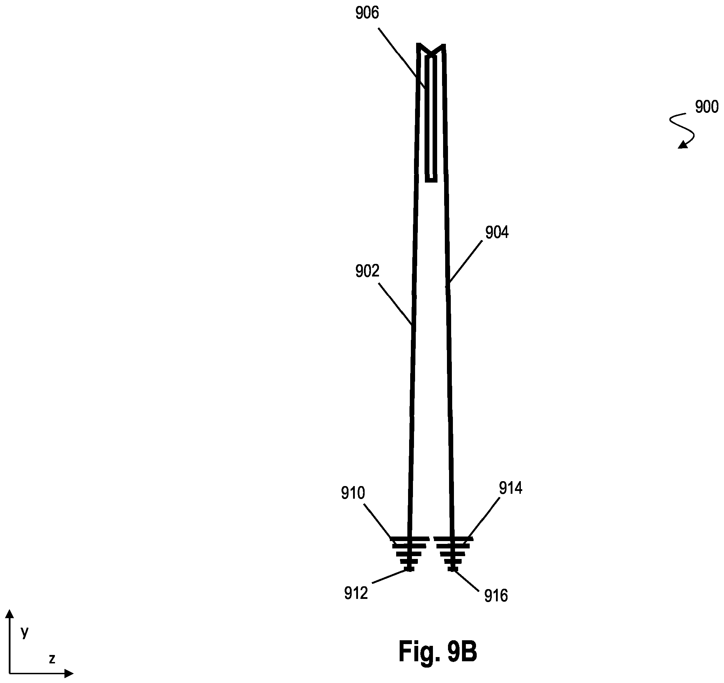

Figs 9A-9B illustrate a diagrammatic view of an implantable clip 900 according to some aspects. Fig. 9A illustrates a front view of the implantable clip 900. For example, the front view may illustrate the implantable clip 900 along an x-y plane such that the x-dimension (i.e. lateral dimension) is oriented in the horizontal direction and the y-dimension (i.e. longitudinal dimension) is oriented in the vertical direction. Fig. 9B illustrates a side view of the implantable clip 900. For example, the side view may illustrate the implantable clip 900 along an y-z plane such that the z-dimension (i.e. depth or thickness dimension) is oriented in the horizontal direction and the y-dimension is oriented in the vertical direction.

The implantable clip 900 may be formed of a single piece of wire, which is bent, curved, or arranged, for example, into a U-shape or V-shape. The implantable clip 900 may include a first arm 902 on a first lateral side of the clip 900 and a second arm 904 on a second lateral side. In some aspects, the first arm 902 and the second arm 904 may be spaced laterally spaced from each other in the x-dimension, as shown by arrow 908. In some aspects, the first arm 902 and the second arm 904 may be generally aligned longitudinally (i.e. in the y-dimension).

A loop 906 may be disposed along the length of wire between the first arm 902 and the second arm 904. The loop 906 may be disposed laterally between the first arm 902 and the second arm 904 such that it is spaced in the x-dimension from both the first arm 902 and the second arm 904. In some aspects, the loop 906 may be longitudinally spaced from the first arm 902 and/or the second arm 904 in the y-dimension. The loop 906 may be formed such that the first arm 902 and the second arm 904 are also spaced along the z-dimension. In some aspects, the loop 906 may allow for flexibility and/or biasing of the implantable clip 900 in one or more dimensions. For example, the first arm 902 and second arm 904 of the clip 900 may be capable of flexing in the x-dimension as shown by arrow 908. Thus, the arms 902, 904 are moveable in the x-dimension, y-dimension, and/or z-dimension.

In some aspects, the clip 900 is formed and/or shaped such that the arms 902, 904 are biased inward towards each other. In some aspects, the clip 900 is flexible and resilient such that the shape of the clip 900 can be non-destructively deformed (e.g., flexed) and the clip 900 will resiliently return to its original shape. In some aspects, the clip 900 can experience a permanent deformation such that the clip 900 is plastically deformed and the arms 902, 904 hold the new shape. The arms 902, 904 may be crimpable such that they can be deformed inward toward each other and hold the new, crimped shape.

The implantable clip 900 may also include a first attachment member 910 on a first end 912 of the first arm 902 and/or a second attachment member 914 on a second end 916 of the second arm 904. The attachment members 910, 914 may allow the clip 900 to engage, secure, and/or attach to either an implanted therapeutic device 890 or the anatomy (i.e. tissue) of the patient. Thus, the first end 912 of the first arm 902 may be implanted into an already-implanted therapeutic device 890 such that the first attachment member 910 attaches to the therapeutic device 890 and prevents the first end 912 from separating from the therapeutic device 890. In some aspects, the first attachment member 910 may attach to a wall 306 of a replacement valve 300. Such as that shown in Figs 3-5B. In some aspects, the first attachment member 910 may attach to the structure 604 and/or mesh 606 of an occlusion device 600, such as that shown in Figs 6-7A. Moreover, the second end 916 of the second arm 904 may be implanted into the anatomy (e.g. cardiac wall 210, LAA 602) such that the second attachment member 914 attaches to the anatomy and prevents the second end 916 from separating from the anatomy.

The attachment members 910, 914 may be any suitable shape, form, or device for attaching to a therapeutic device 890 or anatomy. For example, the attachment members 910, 914 may include barbs, as shown in the illustrated aspects. The barbs may be separate from and attached to the single wire or may be formed therefrom. In another example, the attachment member 910, 914 include one or more hooks. The hooks may be separate from and attached to the single wire or may be formed therefrom. However, the attachment members 910, 914 may also include an adhesive, a roughened surface, or any other member capable of engaging, securing, and/or attaching to a therapeutic device 890 or anatomy. In some aspects, the first and second attachment members 910, 914 may be the same. In other aspects, the first and second attachment members 910, 914 may be different. In some aspects, each end 912, 916 may have multiple attachment members 910, 914.

The single wire may be formed of any suitable material. For example, the wire may be formed of a metal or metal alloy such as stainless steel, nickel, titanium, nitinol, cobalt, chromium, any alloy thereof, or any other suitable metal. In other aspects, the wire may be formed of a polymer such as ultra-high molecular weight polyethylene, polyether ether ketone, shape memory polymers, or any other suitable polymer. The attachment structures 910 may be formed of the same material as the single wire or may be made of a different material. For example, the attachment structures 910 may include barbs formed of any of the metal, metal alloys, or polymers listed above. In some aspects, the barbs may be formed of an absorbable material such as an absorbable sutures.

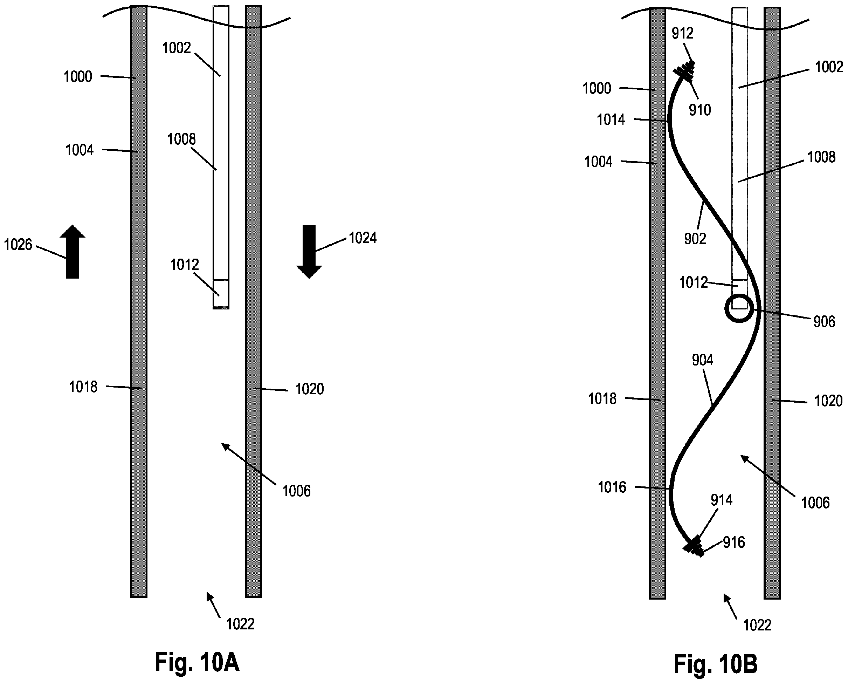

The clip 900 may be delivered to the site of the therapeutic implant 890 and deployed using a clip delivery catheter 1000 and an actuator 1002. Figs 10A-10B illustrate a clip delivery catheter 1000 and an actuator 1002 according to some aspects. Fig. 10A illustrates a cross-sectional view of the clip delivery catheter 1000 and actuator 1002 without the clip 900 and Fig. 10B illustrates the clip delivery catheter 1000 and actuator 1002 with the clip 900. The clip delivery catheter 1000 may include a tube 1004 having a lumen 1006 extending from a proximal end to a distal end of the tube 1004. The distal end comprises a distal opening 1022 through which the clip 900 is pushed or ejected during deployment. The actuator 1002 may be disposed within the lumen 1006 of the tube 1004.

In some aspects, the actuator 1002 may be configured to move and/or push the clip 900 when the clip 900 is disposed within the lumen 1006 of the catheter 1000. For example, the actuator 1002 may be configured to push the clip 900 out of the catheter 1000 and implant the clip 900 into the therapeutic device 890 and/or catheter 1000. Thus, the actuator 1002 may be configured to move distally (as shown by arrow 1024) and/or proximally (as shown by arrow 1026). In some aspects, the actuator 1002 may be configured to hold the clip 900 within the catheter 1000 as the catheter 1000 is moved to the site of the therapeutic implant 890. In some aspects, the actuator 1002 is configured to hold the clip 900 relatively still or steady while the tube 1004 of the catheter 1000 is moved to position the clip 900 outside of the lumen 1006. Thus, the catheter 1000 may be configured to move distally (as shown by arrow 1024) and/or proximally (as shown by arrow 1026). In some aspects, both the actuator 1002 and the catheter 1000 are moveable.

As shown in Fig. 10B, a contact length or coupling structure 1012 may contact the clip 900 at the loop 906. In some aspects, the contact length 1012 may extend through the loop 906 such that the actuator 1002 is releasably coupled to the clip 900.

The clip 900 may be disposed within the lumen 1006 of the catheter 1000 such that the first end 912 points to towards the proximal end of the catheter 1000 and the second end 916 points to the distal end of the catheter 1000. In some aspects, the first end 912 of the first arm 902 is bent outward such that a first bend 1014 is formed in the first arm 902. Similarly, the second end 916 of the second arm 904 is bent outward such that a second bend 1016 is formed in the second arm 904. The first bend 1014 and the second bend 1016 may contact or be disposed proximate to a first side 1018 of the catheter 1000 such that the first bend 1014 and first end 912 of the first arm 902 are spaced proximally from the second bend 1016 and the second end 916 of the second arm 904. The first end 912 is spaced proximally from the first bend 1014 and the second end 916 is spaced distally from the second bend 1016. In some aspects, the loop 906 of the clip 900 may contact or be disposed proximate to a second side 1020 of the catheter 1000 opposite the first side 1018. The walls of the tube 1004 may apply a force to for example, the first bend 1014, the second bend 1016, and/or the loop 906 of the clip 900 to maintain the clip 900 in the illustrated position. In some aspects, the clip 900 is elastically deformed when positioned inside the lumen of the catheter 1006. Thus, the clip 900 may be constrained or flexed such that it is spring-loaded within the lumen 1006 of the catheter 1000.

In some aspects, the clip 900 may be bent such that the first end 912 and second end 916 are pointed in the same direction, either towards the proximal end of the catheter or the distal end. Thus, in some aspects, the arms 902, 904 may be bent inward to fit into the lumen 1006 of the catheter 1000. In this configuration, the first end 912 may contact or be disposed proximate to the first side 1018 of the catheter, the second end 916 may contact or be disposed proximate to the second side 1020 of the catheter 1000, and the loop 906 may be spaced distally or proximally from the ends 912, 916. In this aspect, the ends 912, 916 of the clip 900 may move outside of the catheter 1000 at the same time.

The actuator 1002 may have any suitable shape or form. In some aspects, the actuator 1002 comprises an elongate member 1008 such as a wire, tube, or any other suitable elongate member. The elongate member 1008 may include a stiffness and/or rigidity such that the actuator 1002 can hold and/or move the clip 900.

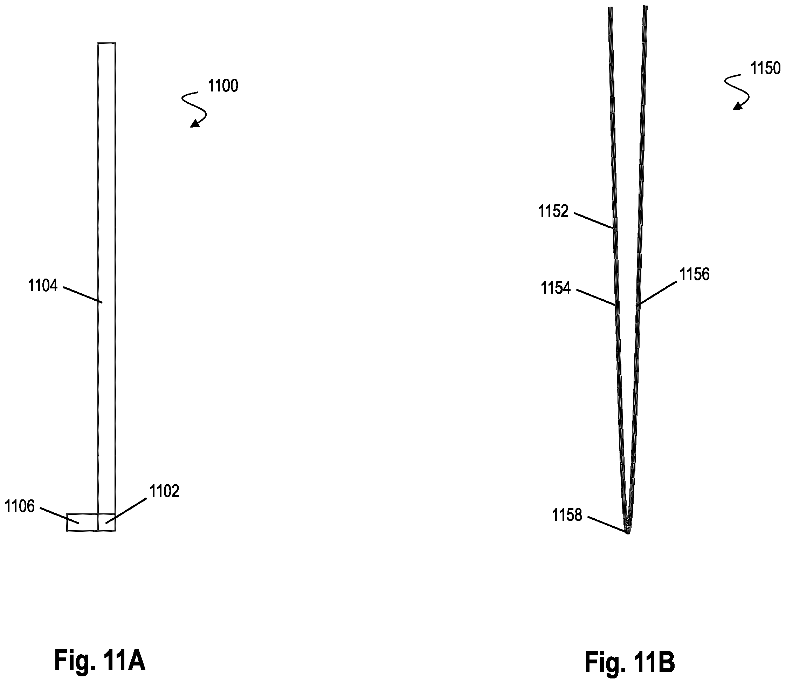

Fig. 11A illustrates a diagrammatic view of one aspect of an actuator 1100 according to some aspects. The distal portion 1102 of the elongate member 1104 may be bent to form the coupling structure 1106. The coupling structure 1106 may be configured to contact the clip 900. In some aspects, the coupling structure 1106 may be disposed at approximately 90 degrees such that the distal portion 1102 of the actuator 1002 forms an L-shape, as shown in Fig. 11A. In some aspects, the coupling structure 1106 may be disposed at an angle less than 90 degrees. In other aspects, the coupling structure 1106 may be disposed at an angle greater than 90 degrees. In some aspects, the coupling structure 1106 may be linear or curved.

Fig. 11B illustrates a diagrammatic view of another aspect of an actuator 1150 according to some aspects. In some aspects, the elongate member 1152 may be bent such that actuator 1150 comprises a first length 1154 on a first side of the bend 1158 and a second length 1156 on a second side of the bend 1158. In this aspect, the first and second length 1154, 1156 may be approximately the same. However, in other aspects, the first and second length 1154, 1156 may be different. In some aspects, the bend 1158 may be the coupling structure 1012 such that the bend 1158 is configured to contact the clip 900.

The actuator 1002 may have any other suitable coupling structure 1012. In some aspects, the distal portion of the actuator 1002 may be threadably coupled to the clip 900. For example, the coupling structure 1012 of the actuator 1002 may include a female/male threaded portion that releasably couples to the other of a female/male threaded portion of the clip 900. In some aspects, the coupling structure 1012 may be a hook, an adhesive, a Velcro-like structure, or any other suitable structure.

The clip delivery catheter 1000 and the actuator 1002 may be used to deliver the implantable clip 900 to the delivery site 1200, which may be the location of the gap 1202 between the therapeutic device 890 and the anatomy 1204. For example, the delivery site may be a gap 410, 420 between a replacement valve 300 and the heart wall 210, as shown in Figs 4A-5B. In another example, the delivery site may be the gap 700 between an occlusion device 600 and the LAA 602, as shown in Figs 7A-7B.

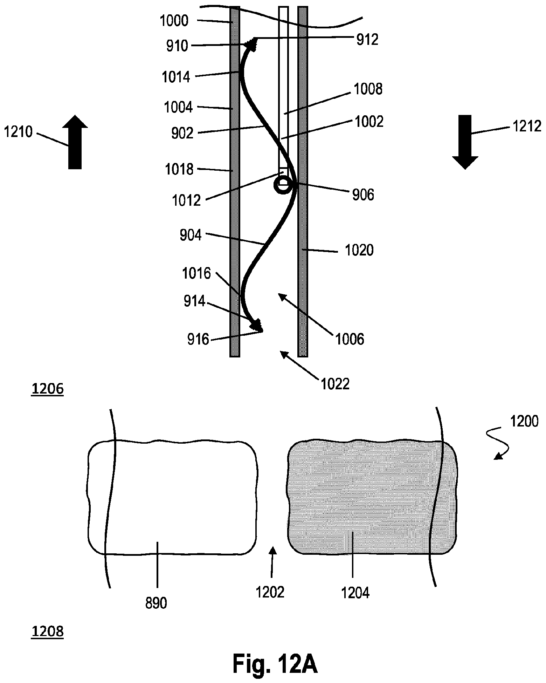

Figs 12A-12G illustrate various steps in a method of deploying and implanting the implantable clip 900. Any suitable clip 900 described herein may be implanted according to the illustrated method, including those described in reference to Figs 9A-9B. Any suitable clip delivery catheter 1000 described herein may be used to deliver and/or deploy the clip 900 according to the illustrated method, including those described in reference to Figs 10A-10B. Any suitable actuator 1002 described herein may be used to deliver and/or deploy the clip 900 according to the illustrated method, including those described in reference to Figs 10A-11B.

Fig. 12A illustrates a diagrammatic view of the clip 900 being delivered to the delivery site 1200. At the delivery site 1200, a gap 1202 may exist between the tissue 1204 and the therapeutic implant 890. A first cardiac volume 1206 may be disposed on a first side (e.g. a first heart chamber, aorta, etc.) of the therapeutic implant 890 and a second cardiac volume 1208 may be disposed on a second side (e.g. a second heart chamber, the left atrial appendage, etc.) of the therapeutic implant 890. In some aspects, the cardiac volumes 1206, 1208 are on either side of a replacement valve 300. For example, normal blood flow through the replacement valve 300 may move from the second cardiac volume 1208 to the first cardiac volume 1206 (in the direction of arrow 1210). In other aspects, the first cardiac volume 1206 may be the left atrium 118 and the second cardiac volume 1208 may be the LAA 602. Thus, the therapeutic implant 890 (e.g. occlusion device 600) may be intended to prevent blood flow from the first cardiac volume 1206 into the second cardiac volume 1208.

In some aspects, the therapeutic implant 890 may have been previously implanted into the patient during a separate procedure. For example, the therapeutic implant 890 may have been implanted between one day and six months before the current method is performed (i.e. the procedure to prevent leakage through the gap 1202). In these aspects, a physician may notice (e.g. during a follow up appointment or check-up) that the therapeutic implant 890 has not been performing properly and/or has a leak. Thus, the physician may decide to perform the current method to treat, plug, and/or close the gap 1202 to improve functioning of the therapeutic implant 890. In some aspects, any degree of endothelialization around the therapeutic implant 890 before the current method is performed such that one or more endothelial cells are disposed on the therapeutic implant 890 and/or tissue 1204.

In other aspects, the therapeutic implant 890 may be implanted during the same procedure as the current method is performed. In these aspects, a physician may notice a leak or gap 1202 between the therapeutic implant 890 and the tissue 1204 after the therapeutic implant 890 has been deployed. Thus, the physician may implement the current methods before the procedure is completed to treat, plug, and/or close the gap 1202 so that the therapeutic implant 890 will perform properly.

As shown in Fig. 12A, the catheter 1000 may be moved proximate to the delivery site 1200 such that the distal opening 1022 is oriented towards the gap 1202. The actuator 1002 may be disposed within the catheter 1000 such that the coupling structure 1012 contacts, engages, and/or holds the loop 906 of the clip 900, as shown in Fig. 10B. The clip 900 may be disposed in the catheter such that the first end 912 of the first arm 902 is pointed proximally (as shown by proximal arrow 1210) relative to the catheter 1000 and the second end 916 of the second arm 904 is pointed distally (as shown by distal arrow 1212) towards the distal opening 1022 of the catheter 1000, as shown in Fig. 10B. As described above, the walls of the tube 1004 of the catheter 1000 may apply a force to for example, the first bend 1014, the second bend 1016, and/or the loop 906 of the clip 900 in this position. Thus, the clip 900 may be constrained or flexed such that it is spring-loaded within the lumen 1006 of the catheter 1000.

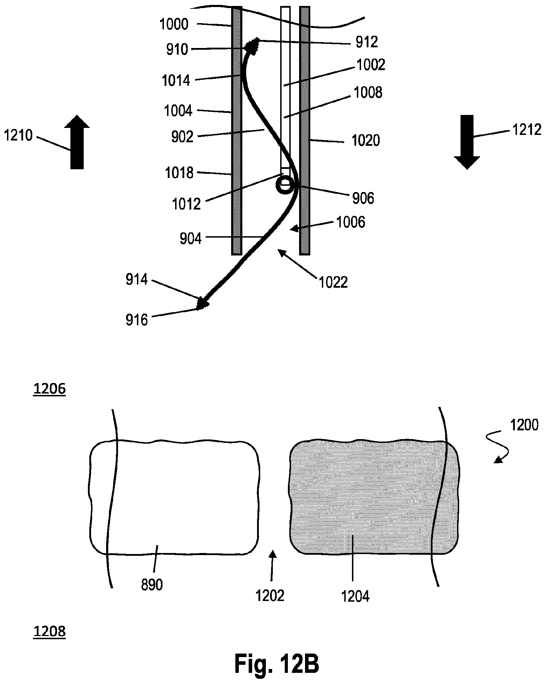

Fig. 12B illustrates a diagrammatic view of the catheter 1000 and/or actuator 1002 moving the second end 916 of the clip 900 out of the distal opening 1022 of the catheter 1000. In some aspects, the actuator 1002 may be moved distally (as shown by arrow 1212) towards the delivery site 1200 to move or push the second end 916 of the clip 900 out of the distal opening 1022 of the catheter 1000. In some aspects, the catheter 1000 may be moved or pulled proximally (as shown by arrow 1210) away from the delivery site 1200 to move the distal opening 1022 over the second end 916 of the clip 900 so that the second end 916 is exposed or moved out of the catheter 1000. In some aspects, the actuator 1002 and catheter 1000 may be moved in coordination such that the second end 916 of the clip 900 out of the distal opening 1022 of the catheter 1000. The actuator 1002 may or may not move out of the distal opening 1022 during this step.

In some aspects, the second end 916 of the clip 900 may be moved out of the distal opening 1022 of the catheter such that the second arm 904 becomes unconstrained by the walls of the tube 1004 of the catheter 1000 such that there is no second bend 1016 in the second arm 904. Thus, the second arm 904 may move inward towards the first arm 902 (e.g., the first arm 902 and the second arm 904 may be biased towards one another). For example, because the clip 900 was elastically deformed when positioned inside the lumen 1006, the clip 900 will return to its original shape when it is outside of the lumen 1006, like the second arm 904 in Fig. 12B.

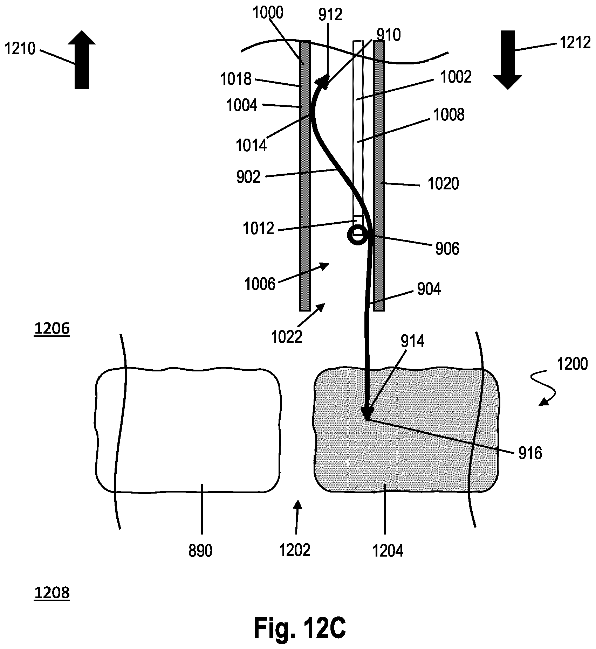

Fig. 12C illustrates a diagrammatic view of the second end 916 of the clip 900 inserted into the tissue 1204. The second attachment member 914 may allow the second end 916 of the clip 900 to engage, secure, and/or attach to the tissue 1204 to prevent the second end 916 from releasing from and/or move out of the tissue 1204.

In some aspects, the catheter 1000 and/or actuator 1002 may be moved to orient the second end 916 of the clip 900 towards the tissue 1204 before it is inserted. The catheter 1000 and/or actuator 1002 may then be moved and/or pushed towards the tissue 1204 (as shown by arrow 1212) with sufficient force such that the second attachment member 914 on the second end 916 of the clip 900 is sufficiently inserted into the tissue 1204. In some aspects, the catheter 1000 and/or actuator 1002 may be moved and/or pulled back to ensure that the second attachment member 914 is sufficiently secured and/or attached to the tissue 1204.

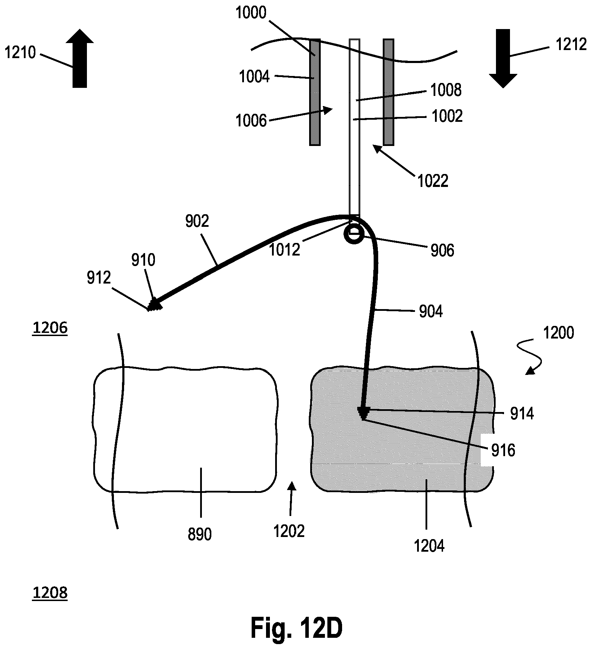

Fig. 12D illustrates a diagrammatic view of the catheter 1000 and/or actuator 1002 moving the first end 912 of the clip 900 out of the distal opening 1022 of the catheter 1000. In some aspects, the actuator 1002 may be moved distally (as shown by arrow 1212) towards the delivery site 1200 to move or push the first end 912 of the clip 900 out of the distal opening 1022 of the catheter 1000. In some aspects, the catheter 1000 may be moved or pulled proximally (as shown by arrow 1210) away from the delivery site 1200 to move the distal opening 1022 over the first end 912 of the clip 900 so that the first end 912 is exposed or moved out of the catheter 1000. In some aspects, the actuator 1002 and catheter 1000 may be moved in coordination such that the first end 912 of the clip 900 out of the distal opening 1022 of the catheter 1000. The actuator 1002 may move out of the distal opening 1022 during this step.

In some aspects, the first end 912 of the clip 900 may be moved out of the distal opening 1022 of the catheter such that the first arm 902 becomes unconstrained by the walls of the tube 1004 of the catheter 1000 such that there is no first bend 1014 in the first arm 902. For example, because the clip 900 was elastically deformed when positioned inside the lumen 1006, the clip 900 will return to its original shape when it is outside of the lumen 1006, like the first arm 902 in Fig. 12D. Thus, the first arm 902 may move or inward towards the second arm 904 (e.g., the first arm 902 and the second arm 904 may be biased towards one another).

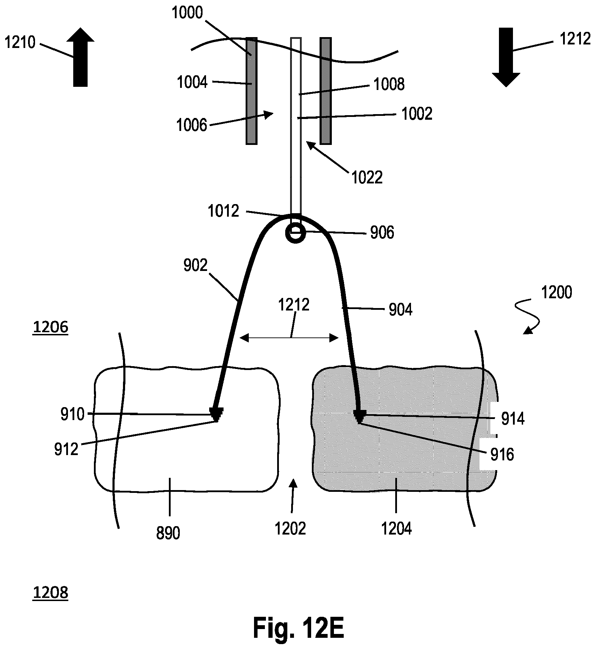

Fig. 12E illustrates a diagrammatic view of the first end 912 of the clip 900 inserted into the therapeutic implant 890. The first attachment member 910 may allow the first end 912 of the clip 900 to engage, secure, and/or attach to the therapeutic implant 890 to prevent the first end 912 from releasing from and/or move out of the therapeutic implant 890.

In some aspects, the catheter 1000 and/or actuator 1002 may be moved to orient the first end 912 of the clip 900 towards the therapeutic implant 890 before it is inserted. The catheter 1000 and/or actuator 1002 may then be moved and/or pushed towards the therapeutic implant 890 (as shown by arrow 1212) with sufficient force such that the first attachment member 910 on the first end 912 of the clip 900 is sufficiently inserted into the therapeutic implant 890. In some aspects, the catheter 1000 and/or actuator 1002 may be moved and/or pulled back to ensure that the first attachment member 910 is sufficiently secured and/or attached to the therapeutic implant 890.

When the first attachment member 910 attaches the first end 912 to the therapeutic implant 890, the arms 902, 904 are a first distance 1214 away from each other. The clip 900 may extend across the gap 1202 such that the first distance 1214 includes the width of the gap 1202.

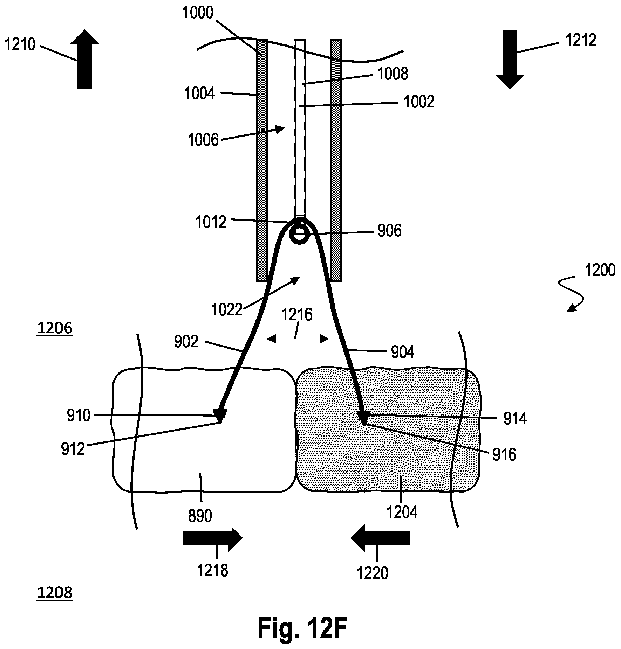

Fig. 12F illustrates a diagrammatic view of the catheter 1000 and/or actuator 1002 moving the therapeutic implant 890 and tissue 1204 towards each other by moving the first arm 902 and second arm 904 of the clip 900 towards each other. In some aspects, the actuator 1002 may be moved proximally (as shown by arrow 1210) away from the delivery site 1200 to move or pull the loop 906 of the clip 900 upward. In some aspects, the catheter 1000 may be moved or pulled distally (as shown by arrow 1212) towards the delivery site 1200 to contact and move the first arm 902 and second arm 904 closer to each other. In some aspects, the actuator 1002 and catheter 1000 may be moved in coordination such that the first arm 902 and second arm 904 towards each other.

The actuator 1002 is moved proximally into the catheter 1000 such that the first side 1018 of the catheter 1000 contacts and applies a force to the first arm 902 and the second side 1020 of the catheter 1000 contacts and applies a force to the second arm 904. This force applied by the catheter 1000 presses the arms 902, 904 towards each other. As the arms 902, 904 are pressed inward by the catheter 1000, the arms 902, 904 press the therapeutic device 890 and the tissue 1204 towards each other (as shown by arrows 1218, 1220) until the therapeutic implant 890 and the tissue 1204 contact each other or are disposed proximate each other. In some aspects, this may close the gap 1202 at the delivery site 1200. In some aspects, the arms 902, 904 may include an inward bend from the force of the catheter 1000 on the arms 902, 904. Thus, the arms 902, 904 may be crimped or plastically deformed such that they maintain the new shape once the catheter 1000 and/or actuator 1002 are removed as long as the force acting to move the arms 902, 904 outward from each other is not great enough to deform the arms 902, 904 outward again.

When the arms 902, 904 are forced together by the catheter 1000, the arms 902, 904 are a second distance 1216 away from each other. The second distance 1216 is smaller than the first distance 1214 shown in Fig. 12E. In some aspects, the second distance 1216 does not include the width of the gap 1202, because the arms 902, 904 have been forced together to close the gap 1202.

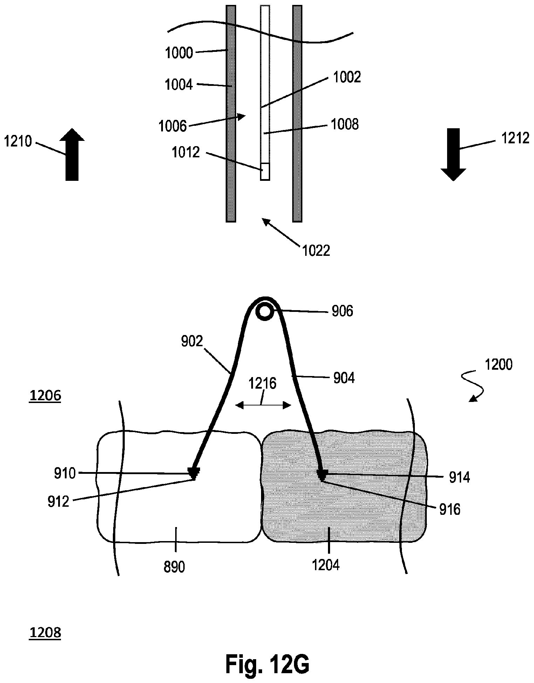

Fig. 12G illustrates a diagrammatic view of the actuator 1002 and catheter 1000 disengaging from the clip 900 by releasing the loop 906. In some aspects, the coupling element 1012 of the actuator 1002 may disengage or uncouple from the clip 900 in any suitable way. For example, when the coupling structure 1012 includes an L-shape 1106 as shown in Fig. 11A, the distal portion 1102 of the actuator 1100 may be moved such that the horizontal portion of the L-shape 1106 moves away from the loop 906. Thus, in Fig. 12G, the actuator 1100 with the L-shaped coupling structure 1106 may be moved backward (i.e. into the page) to disengage the loop 906.

In another example, when the coupling structure 1012 is a bend 1158 in the elongate member 1152 as shown in Fig. 11B, the actuator 1150 may be removed from the loop 906 by pulling on or distally moving the first length 1154 on the first side of the bend 1158. Thus, as the first length 1154 is pulled distally, the second length 1156 on the opposite side of the bend 1158 is pulled through the loop 906 until the entire second length 1156 is pulled through. The bend 1158 may be straightened out during this process to disengage the loop 906 before the second length 1156 is pulled through.

In yet another example, when the coupling structure 1012 of the actuator 1002 is threaded to a structure on the clip 900, the coupling structure 1012 may be unthreaded from the clip 900 to disengage or decouple from it. In another example, when the coupling structure 1012 of the actuator 1002 is a hook, the actuator 1002 may be moved such that the hook is unhooked from the clip 900. However, other coupling structures 1012 and ways of disengaging, decoupling, and/or releasing the clip 900 are contemplated.

Once the actuator 1002 disengages, decouples, and/or releases the clip 900, the catheter 1000 and actuator 1002 may be moved away from the delivery site 1200 (as shown by arrow 1210). In some aspects, the actuator 1002 may be moved proximally and may be removed from the catheter 1000 so that a new clip 900 can be engaged with the actuator 1002 and inserted into the catheter 1000. Thus, another clip 900 can be delivered to and deployed at the current delivery site 1200 or a new delivery site along the therapeutic implant 890. Once all of the desired clips 900 have been deployed, the catheter 1000 and actuator 1002 may be removed from the patient. In other aspects, the catheter 1000 and actuator 1002 may be removed completely and a new catheter 1000, actuator 1002, and clip 900 may be inserted into the patient to deliver subsequent clips 900.

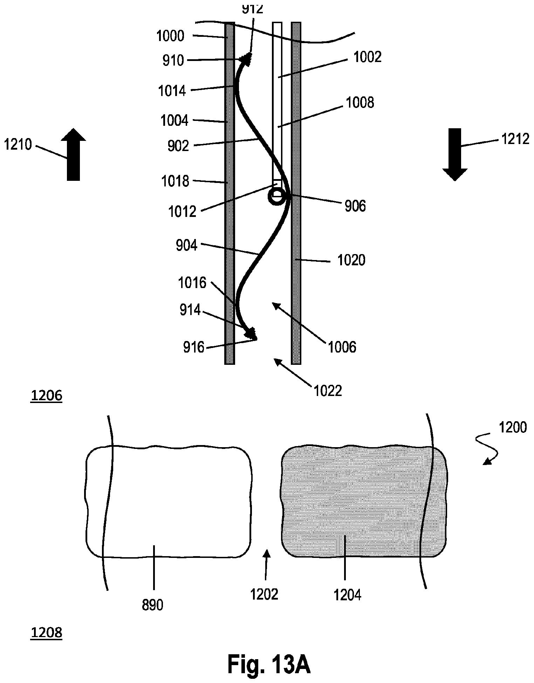

Figs 13A-13E illustrate various steps in another aspect of a method of delivering and implanting the clip 900 at a delivery site 1200 according to some aspects. This method is largely similar to the method illustrated in Figs 12A-12G. However, in the method illustrated in Figs 13A-13E, the ends 912, 916 are implanted into the therapeutic implant 890 and the tissue 1204 at the same time instead of implanting one end 912, 916 at a time.

Fig. 13A illustrates a diagrammatic view of the clip 900 being delivered to the delivery site 1200. This step is identical to the step illustrated in Fig. 12A.

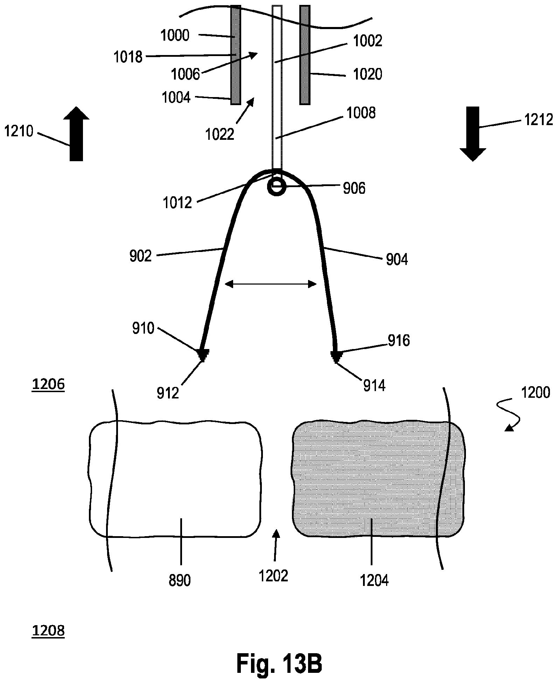

Fig. 13B illustrates a diagrammatic view of the catheter 1000 and/or actuator 1002 moving both the first end 912 and the second end 916 of the clip 900 out of the distal opening 1022 of the catheter 1000. In some aspects, this may be accomplished by performing the step shown in Fig. 12B, then immediately performing the step shown in Fig. 12D.

In some aspects, the actuator 1002 may be moved distally (as shown by arrow 1212) towards the delivery site 1200 to move or push the second end 916 of the clip 900 out of the distal opening 1022 of the catheter 1000. The actuator 1002 may then continue to be moved distally towards the delivery site 1200 to move or push the first end 912 of the clip 900 out of the distal opening 1022. In some aspects, the catheter 1000 may be moved or pulled proximally (as shown by arrow 1210) away from the delivery site 1200 to move the distal opening 1022 over the second end 916 of the clip 900 so that the second end 916 is exposed or moved out of the catheter 1000. The catheter 1000 may then continue to be moved or pulled proximally away from the delivery site 1200 to move the distal opening 1022 over the first end 912 of the clip 900 so that the first end 912 is exposed or moved out of the catheter 1000. In some aspects, the actuator 1002 and catheter 1000 may be moved in coordination such that the second end 916 of the clip 900 is moved out of the distal opening 1022 of the catheter 1000, followed by the first end 912. The actuator 1002 may move out of the distal opening 1022 during this step.

In some aspects, the ends 912, 916 of the clip 900 may be moved out of the distal opening 1022 of the catheter such that the arms 902, 904 become unconstrained by the walls of the tube 1004 of the catheter 1000. Thus, when the arms 902, 904 are uncontrained, there may not be a first bend 1014 or a second bend 1016 in the first arm 902 or second arm 904, respectively. For example, because the clip 900 was elastically deformed when positioned inside the lumen 1006, the clip 900 will return to its original shape when it is outside of the lumen 1006, like the first arm 902 and the second arm 904 in Fig. 12B. Therefore, the arms 902, 904 may move inward towards each other (e.g., the first arm 902 and the second arm 904 may be biased towards one another). In some aspects, the clip 900 returns to its original position illustrated in, for example, Figs 9A-9B.

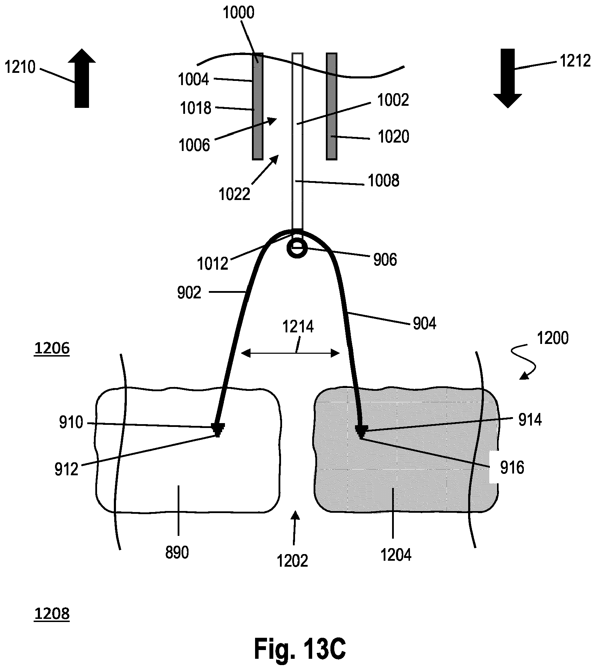

Fig. 13C illustrates a diagrammatic view of the first end 912 and second end 916 of the clip 900 inserted into the therapeutic implant 890 and the tissue 1204, respectively. The first attachment member 910 may allow the first end 912 of the clip 900 to engage, secure, and/or attach to the therapeutic implant 890 to prevent the first end 912 from releasing from and/or move out of the therapeutic implant 890. Similarly, the second attachment member 914 may allow the second end 916 of the clip 900 to engage, secure, and/or attach to the tissue 1204 to prevent the second end 916 from releasing from and/or move out of the tissue 1204.

In some aspects, the catheter 1000 and/or actuator 1002 may be moved to orient the first end 912 of the clip 900 towards the therapeutic implant 890 and the second end 916 of the clip 900 towards the tissue 1204 before they are inserted. In some aspects, if the arms are not oriented to enter the therapeutic implant 890 or tissue 1204 properly (e.g., arms are not parallel enough or perpendicular enough to tissue/therapeutic implant), then the actuator 1002 can pull or move the clip 900 back proximally so that the catheter wall 1000 urges the arms 902, 904 towards one another before being inserted into the therapeutic implant 890 or tissue 1204. Thus, the arms 902, 904 may form a V-shape before they are inserted.

The catheter 1000 and/or actuator 1002 may then be moved and/or pushed towards the delivery site 1200 (as shown by arrow 1212) with sufficient force such that the first attachment member 910 on the first end 912 of the clip 900 is sufficiently inserted into the therapeutic implant 890 and the second attachment member 914 on the second end 916 of the clip 900 is sufficiently inserted into the tissue 1204. In some aspects, the catheter 1000 and/or actuator 1002 may be moved and/or pulled back to ensure that the attachment members 910, 914 are sufficiently secured and/or attached to the therapeutic implant 890 and tissue 1204.

When the attachment members 910, 914 attach the ends 912, 916 of the clip 900 to the therapeutic implant 890 and the tissue 1204, the arms 902, 904 are a first distance 1214 away from each other. The clip 900 may extend across the gap 1202 such that the first distance 1214 includes the width of the gap 1202.

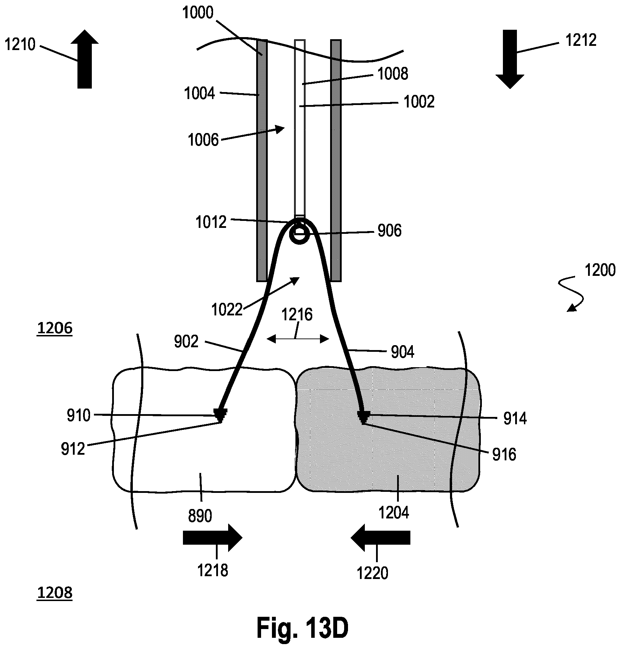

Fig. 13D illustrates a diagrammatic view of the catheter 1000 and/or actuator 1002 moving the therapeutic implant 890 and tissue 1204 towards each other by moving the first arm 902 and second arm 904 of the clip 900 towards each other, thereby closing the gap 1202. This step is identical to the step illustrated in Fig. 12F.

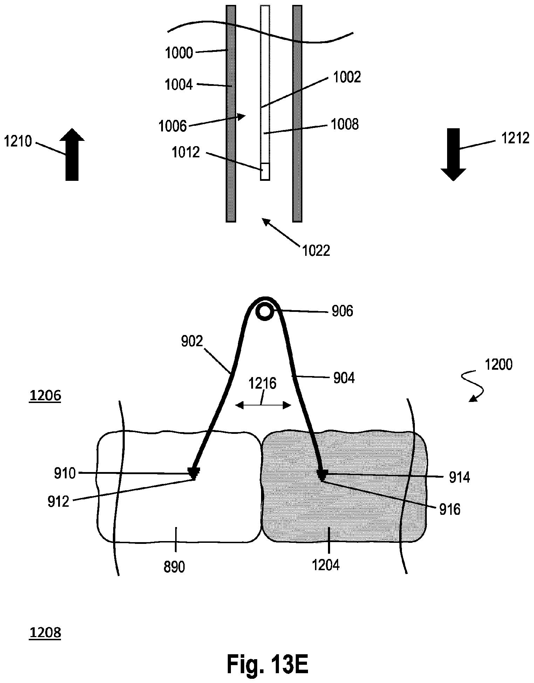

Fig. 13E illustrates a diagrammatic view of the actuator 1002 and catheter 1000 disengaging from the clip 900 by releasing the loop 906. This step is identical to the step illustrated in Fig. 12G.

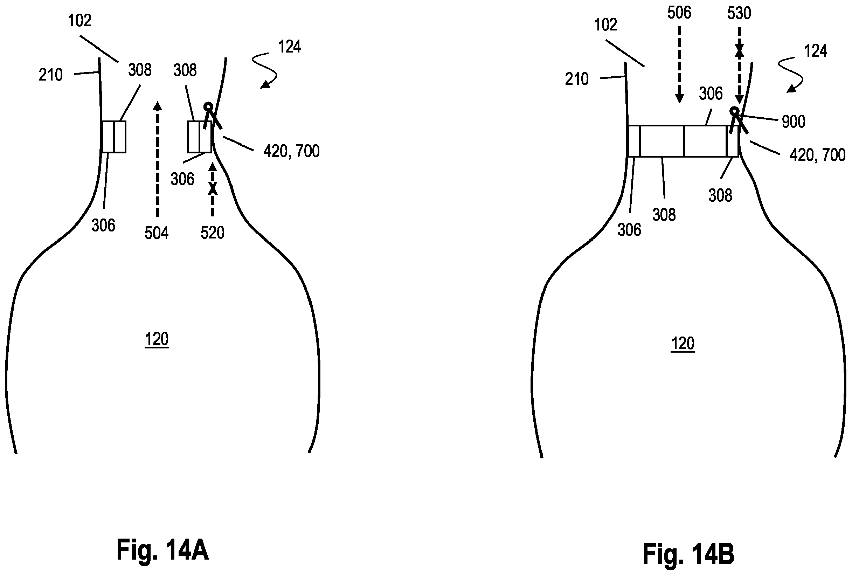

Figs 14A-14B illustrate a diagrammatic cross-sectional side view of a replacement valve 300 shown in Figs 5A-5B after a gap has been closed using one or more clips 900. Fig. 14A illustrates the replacement valve 300 with the valve leaflets 308 open to allow blood to flow from the left ventricle 120 to the aorta 102 (i.e. during systole), indicated by directional arrow 504. Because the clip 900 has been implanted into the replacement valve 300 and the heart wall 210 such that the replacement valve 300 and the heart wall 210 are in contact, there is no gap between the replacement valve 300 and the heart wall 210. Thus, during systole, blood flows through the opening of the replacement valve 300 (as shown by arrow 504) but there is no blood flow around the outside of the replacement valve 300 along the heart wall 210 (as shown by arrow 520).

Fig. 14B illustrates the replacement valve 300 with the valve leaflets 308 closed (i.e.
during diastole) to prevent mitral regurgitation (i.e. blood flow from the aorta 102 to the left ventricle as shown by arrow 506). Because there is no gap between the replacement valve 300 and the heart wall 210, there is no blood flow between the outside of the replacement valve 300 and the heart wall 210 (as shown by arrow 530). Therefore, the deployment of the clip 900 may prevent paravalvular leakage around the replacement valve 300, which may allow the valve to function properly.

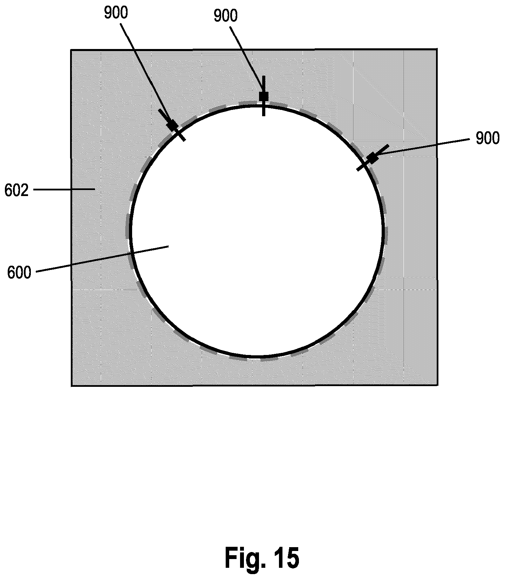

Fig. 15 illustrates a diagrammatic top view of an occlusion device 600 shown in Fig. 7C after a gap has been closed using one or more clips 900 according to aspects of the present disclosure. In the illustrated aspects, three clips 900 have been implanted into the occlusion device 600 and LAA 602. However, any suitable number of clips 900 may be implanted. For example, 1, 2, 4, 5, 6, 7, 8, 9, or 10 clips 900 may be implanted to close one or more gaps between the occlusion device 600 and the wall of the LAA 602. In some aspects, when multiple clips 900 have been implanted, the clips 900 may be evenly spaced along the gap. In other aspects, the clips 900 may be unevenly spaced along the gap. When one or more clips 900 have been implanted to close the gap, there may be no blood flow from the left artery into the LAA 602.

Fig. 16 is a schematic diagram of a processor circuit 1600, according to aspects of the present disclosure. The processor circuit 1600 may be implemented in the processing system 810, the system 800, or other devices or workstations (e.g., third-party workstations, network routers, etc.), or on a cloud processor or other remote processing unit, as necessary to implement the method. As shown, the processor circuit 1600 may include a processor 1610, a memory 1612, and a communication module 1614. These elements may be in direct or indirect communication with each other, for example via one or more buses.

The processor 1610 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1610 may also comprise another hardware device, a firmware device, or any combination thereof configured to perform the operations described herein. The processor 1610 may also be implemented as a combination of computing devices, e.g., a combination of a DSP and a microprocessor, a plurality of microprocessors, one or more microprocessors in conjunction with a DSP core, or any other such configuration.

The memory 1612 may include a cache memory (e.g., a cache memory of the processor 1610), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 1612 includes a non-transitory computer-readable medium. The memory 1612 may store instructions 1616. The instructions 1616 may include instructions that, when executed by the processor 1610, cause the processor 1610 to perform the operations described herein. Instructions 1616 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1614 can include any electronic circuitry and/or logic circuitry to facilitate direct or indirect communication of data between the processor circuit 1600, and other processors or devices. In that regard, the communication module 1614 can be an input/output (I/O) device. In some instances, the communication module 1614 facilitates direct or indirect communication between various elements of the processor circuit 1600 and/or the system 800. The communication module 1614 may communicate within the processor circuit 1600 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the leaflet puncture and slitting device) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles), 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

As will be readily appreciated by those having ordinary skill in the art after becoming familiar with the teachings herein, the clip 900 advantageously permits a clinician (e.g., a heart surgeon) to reversibly repair leaks that may form during a therapeutic implant 890 (e.g. a replacement valve 300 or an occlusion device 600). Although primarily intended for the repair of leakage of therapeutic implants 890 for the heart, the same technology could be applied to any implant or device where leakage occurs.

The logical operations making up the aspects of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of aspects of the present disclosure. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the present disclosure, e.g., as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. An apparatus, comprising:
an implantable clip comprising a single length of wire, wherein the single length of wire is shaped into:
a first arm;
a second arm; and
a loop positioned laterally between the first arm and the second arm and longitudinally proximal of the first arm and the second arm,
wherein the first arm is configured to be inserted into a therapeutic implant within a heart of a patient and the second arm is configured to be inserted into a native tissue of the heart,
wherein the first arm and the second arm are configured to urge a border of the therapeutic implant and a border of the native tissue toward one another and close a gap between the therapeutic implant and the native tissue.

2. The apparatus of claim 1, wherein the first arm comprises a barbed first end that is inserted into the therapeutic implant and the second arm comprises a barbed second end that is inserted into the native tissue.

3. The apparatus of claim 1, further comprising:
a delivery catheter comprising a lumen configured to receive the implantable clip before the first arm is inserted into the therapeutic implant and the second arm is inserted into the native tissue of the heart.

4. The apparatus of claim 3, wherein, when the implantable clip is positioned within the lumen:
the first arm comprises a first bend on a first side of the loop and the second bend on a second side of the loop; and
the first bend and the second bend are proximate to a first side of the delivery catheter and the loop is proximate to an opposite second side of the delivery catheter.

5. The apparatus of claim 4,
wherein the first arm comprises a barbed first end proximal of the first bend and the second arm comprises a barbed second end distal of the second bend,
wherein, when the implantable clip is positioned within the lumen, the barbed first end is proximal-facing and the barbed second end is distal-facing.

6. The apparatus of claim 3, further comprising an actuator positioned within the lumen and configured to be removably coupled to the implantable clip.

7. The apparatus of claim 6, wherein the actuator is shaped to be received through the loop to removably couple the actuator and the implantable clip.

8. The apparatus of claim 6,
wherein the delivery catheter comprises a catheter wall defining an opening at a distal end of the lumen,
wherein the actuator is configured to provide distal motion to the implantable clip to move the implantable clip outside of the lumen.

9. The apparatus of claim 8,
wherein the actuator is configured to provide proximal motion to the implantable clip, while the implantable clip is positioned outside of the lumen, such that the first and second arm contact the catheter wall, and
wherein, in response to the proximal motion, the catheter wall is configured to urge the first arm and the second arm towards one another while the first arm is inserted into the therapeutic implant and the second arm is inserted into the native tissue such that the border of the therapeutic implant and the border of the native tissue are urged toward one another.

10. A system for treating leakage between a therapeutic implant and tissue of a patient, comprising:
a catheter comprising a lumen, a distal portion, and a proximal portion; and
the apparatus according to any of claims 1-9,
wherein the implantable clip is disposed in the lumen of the catheter at the distal portion of the catheter before the implantable clip is implanted in the patient, wherein the implantable clip comprises a first wire comprising the first arm and the second arm,
wherein the first arm comprises a first attachment member configured to be inserted into the therapeutic implant, and
wherein the second arm comprises a second attachment member configured to be inserted into the native tissue of the heart.

11. The system of claim 10, wherein at least one of the first attachment member or the second attachment member comprises a hook.

12. The system of claim 10, wherein at least one of the first attachment member or the second attachment member comprises a plurality of barbs.

13. The system of claim 10, wherein the actuator comprises a second wire comprising a distal end and a bend proximate the distal end, wherein the bend contacts the implantable clip.

14. The system of claim 13, wherein the bend in the second wire is approximately 90 degrees such that the second wire is L-shaped at the distal end.

15. The system of claim 10, wherein the first wire comprises the loop between the first arm and the second arm.
